# EUROPÄISCHE PATENTSCHRIFT

(11) **EP 1 119 383 B1**
(45) Veröffentlichungstag und Bekanntmachung des Hinweises auf die Patenterteilung: **23.06.2004**
(21) Anmeldenummer: 99942908.7
(22) Anmeldetag: 24.08.1999
(51) Int. Cl.: A61M 15/00

(54) **DOSIERAEROSOLABGABEVORRICHTUNG MIT EINER BETÄTIGUNGSEINRICHTUNG FÜR ZÄHLWERKE**
METERING AEROSOL DISPENSING DEVICE WITH AN ACTUATING DEVICE FOR METERS
DISPOSITIF DOSEUR DISTRIBUTEUR D'AEROSOL DOTE D'UN MOYEN D'ACTIONNEMENT POUR DES DISPOSITIFS COMPTEURS

(30) Priorität: 08.10.1998 DE 19846382
(43) Veröffentlichungstag der Anmeldung: 01.08.2001
(73) Patentinhaber: PARI GmbH Spezialisten für effektive Inhalation, 82319 Starnberg (DE)
(72) Erfinder: GALLEM, Thomas, D-81369 München (DE); STAPLETON, Kevin, Brookline, MA 02446-3011 (US); KNOCH, Martin, D-82335 Berg (DE)
(74) Vertreter: HOFFMANN - EITLE
(86) Internationale Anmeldenummer: PCT/EP1999/006205
(87) Internationale Veröffentlichungsnummer: WO 2000/021593

(56) Entgegenhaltungen:
- FR-A- 2 022 212
- GB-A- 1 317 315
- US-A- 5 349 945
- US-A- 5 482 030

## Beschreibung

Die vorliegende Erfindung betrifft Dosieraerosolabgabevorrichtungen, und insbesondere eine Betätigungsvorrichtug für Zählwerke zur einfachen Verwendung in Dosieraerosolabgabevorrichtungen.

Verschiedene Dosieraerosolabgabevorrichtungen für Inhalationszwecke sind bekannt, die beispielsweise zur Behandlung von Atemwegserkrankungen eine dosisgenaue Medikamentenmenge in Form eines Sprühnebels oder Aerosols abgeben. Hierbei kommt es insbesondere auf die zuverlässige Dosierung des abgegebenen Medikaments an, damit ein gezielter Therapieeffekt beim Patienten hervorgerufen wird. Derartige Dosieraerosolvorrichtungen sind beispielsweise die allgemein bekannten MDI (metered dose inhalers), die in der Regel ein Gehäuse, einen Behälter zur Aufnahme eines flüssigen oder pulverförmigen Medikaments, eine geeignet ausgebildete Düse zum Zerstäuben und Verteilen des Medikaments und ein Mundstück aufweisen, über das das erzeugte medikamentenhaltige Aerosol eingeatmet wird. Die Betätigung der Dosieraerosolvorrichtungen erfolgt beispielsweise bei Treibgasaerosolen derart, daß der Medikamentenbehälter gegenüber der in dem Gehäuse angeordneten Düse geradlinig verschoben wird, wodurch eine definierte Menge des Zerstäubungsguts freigesetzt wird. Eine Dosieraerosolvorrichtung dieser Art ist beispielsweise in EP 0 254 391 beschrieben.

Dosieraerosolabgabevorrichtungen sind in der Regel für Mehrfachdosierungen ausgelegt. Dabei ist es wünschenswert, dem Benutzer die Anzahl der ausgegebenen Dosen, in anderen Worten der abgegebenen Sprühstöße, bzw. die. noch zur Verfügung stehenden Sprühstöße anzuzeigen, um sicher zustellen, daß der Patient frühzeitig darauf hingewiesen wird, daß das Medikament verbraucht ist. Auf diese Weise wird vermieden, daß ein Patient, beispielsweise zur Vorbeugung eines akuten Asthmaanfalls, eine bereits nahezu leere Dosieraerosolabgabevorrichtung mit sich führt.

Aus diesem Grund sind Dosiervernebler bzw. Dosierinhalatoren mit unterschiedlichen Zählwerken ausgestattet worden.
EP 0 254 391 beschreibt zum Beispiel einen Inhalator mit einem flachen Zählwerk, das auf der dem Patienten zugewandten Seite eines Aerosolspenders ausgebildet ist. Aus EP 0 505 321 ist ein wiederverwendbarer Inhalator mit rücksetztbarem Zähler bekannt, der beim Erreichen der ersten Relativposition von Vorratskammer und Dosierstift inkrementiert wird. Das in GB 1 317 315 offenbarte Zählwerk weist eine Vielzahl von ringförmigen Elementen auf, die mechanisch zusammenwirken, um an einem Anzeigering die noch zur Verfügung stehenden Dosierungen anzuzeigen. Weitere Medikamentenspender mit mechanischem Zählwerk sind beispielsweise in FR 2.022.212, WO 86/02275 und WO 93/24167 offenbart.

Die bekannten Lösungen für Dosierungsvorrichtungen weisen jedoch entweder einen komplexen, unhandlichen Aufbau auf oder erfordern, dass eine bereits vorhandene Dosisaerosolabgabevorrichtung in erheblichem Umfang modifiziert werden muss. Eine derartige Modifikation einer bereits vorhandenen Dosieraerosolabgabevorrichtung ist jedoch insofern von Nachteil, da diese dann erneut einem amtlichen Genehmigungsverfahren und den damit einhergehenden Versuchen für die medizinische Anwendbarkeit unterworfen werden muss. Dies ist jedoch in der Regel ein langwieriger und kostspieliger Prozess und daher unerwünscht.

Ausgehend vom Stand der Technik ist es daher die Aufgabe der vorliegenden Erfindung, für eine Dosieraerosolvorrichtung eine Betätigungsvorrichtung für Zählwerke mit kleinen Abmessungen zu schaffen, die mit unterschiedlichen Dosieraerosolabgabevorrichtungen zur Dosiszählung verwendet werden kann, ohne dass wesentliche Modifikationen an den Dosieraerosolvorrichtungeh durchgeführt werden müssen.

Die Aufgabe wird erfindungsgemäß gelöst durch eine Dosieraerosolabgabevorrichtung mit einem Aerosolbehälter, einer in einem Düsenkörper angeordneten Düse, über die ein Aerosol abgegeben wird, wenn der Aerosolbehälter gegenüber dem Düsenkörper bei einer Betätigung der Dosieraerosolabgabevorrichtung verschoben wird, einem Zählwerk zur Zählung der Betätigungen der Dosieraerosolabgabevorrichtung und einer Betätigungsvorrichtung zur Weiterschaltung des Zählwerks bei einer Betätigung der Dosieraerosolabgabevorrichtung, wobei die Betätigungsvorrichtung zwei gegeneinander verschiebbare Hülsen umfasst, die im Inneren den Düsenkörper der Dosieraerosolabgabevorrichtung derart aufnehmen, dass die Düse zur unbeeinflussten Aerosolerzeugung freigegeben ist, wobei die Hülsen geradlinig gegeneinander verschiebbar sind und wobei die Hülsen durch Federelemente miteinander verbunden sind, die die Hülsen in eine Ausgangsposition zurück bewegen.

Die Federelemente sind vorteilhaft in Form von flachen, gebogenen Federelementen realisiert , die an den Außenseiten der Hülsen befestigt oder einstückig angeformt sind.

Um eine Verkantung der Hülsen zu vermeiden, sind an den Hülsen der Betätigungsvorrichtung Führungselemente vorgesehen sind, die eine geradlinige Verschiebung der beiden Hülsen gegeneinander gewährleisten. Alternativ können die Federelemente derart ausgestaltet werden, dass sie eine geradlinige Verschiebung der beiden Hülsen gegeneinander gewährleisten.

Für die Weiterschaltung des Zählwerks umfasst die Betätigungsvorrichtung einen Betätigungsarm umfasst, der auf das Zählwerk einwirkt.

Um ein möglichst kleines Zählwerk zu realisiern, besteht das Zählwerk aus mehreren parallel angeordneten Scheibeneinheiten wobei eine erste Scheibeneinheit eine Außenverzahnung aufweist, die mit der Betätigungsvorrichtung, insbesondere mit dem Betätigungsarm zusammenwirkt.

Um aus Zählwerk und Betätigungsvorrichtung eine Einheit zu machen, weist die Betätigungsvorrichtung eine Öffnung auf, in der ein Lagerbolzen des Zählwerks zur Halterung des Zählwerks an der Betätigungsvorrichtung befestigt ist.

Damit die Aerosolerzeugung und -ausbreitung nicht beeinflusst wird, weist die Betätigungseinrichtung, insbesondere das Unterteil eine Aussparung oder eine Öffnung auf, durch die die Düse freigegeben ist.

Weitere Merkmale und Vorteile der vorliegenden Erfindung ergeben sich aus der folgenden Beschreibung eines bevorzugten Ausführungsbeispieles, das im Zusammenhang mit den beigefügten Abbildungen erläutert wird, in denen zeigt:
- Fig. 1: eine Explosionsdarstellung eines Zählwerks, das mit einer erfindungsgemäßen Betätigungsvorrichtung für eine Dosieraerosolabgabevorrichtung zusammenwirkt;
- Fig. 2: eine Explosionsdarstellung des in Fig. 1 gezeigten Zählwerks, betrachtet aus der zu Fig. 1 anderen Blickrichtung;
- Fig. 3: eine schematische Querschnittsansicht einer Ausgangs- bzw. Ruheposition der zweiten und dritten Scheibeneinheiten zur Erläuterung des Zusammenwirkens von zweiter und dritter Scheibeneinheit;
- Fig. 4: eine schematische Querschnittsansicht analog zu Fig.3, die die zweite und dritte Scheibeneinheit in einer Position zeigt, in der die zweite Scheibeneinheit durch die dritte Scheibeneinheit veranlaßt wird, mit der vierten Scheibeneinheit zusammenzuwirken;
- Fig. 5: eine separate Ansicht der in Fig. 1 und 2 dargestellten Betätigungsvorrichtung;
- Fig. 6: die Anordnung der erfindungsgemäßen Betätigungseinrichtung für Zählwerke in einer Dosieraerosolabgabevorrichtung; und
- Fig. 7: eine vergrößerte Darstellung der Anordnung der Betätigungseinrichtung an der Düse einer Dosieraerosolabgabevorrichtung.

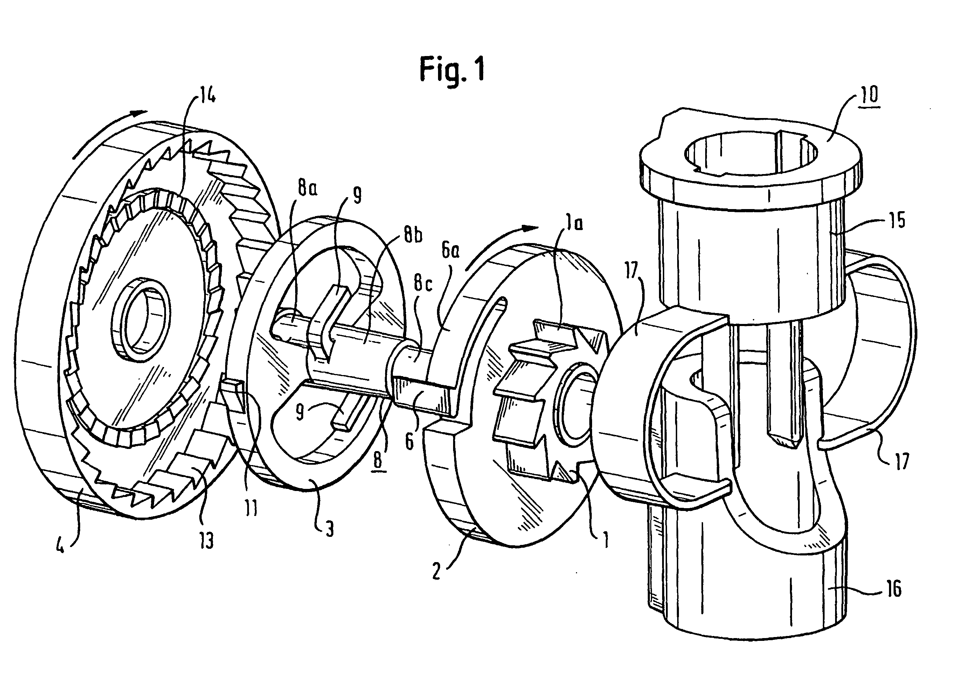

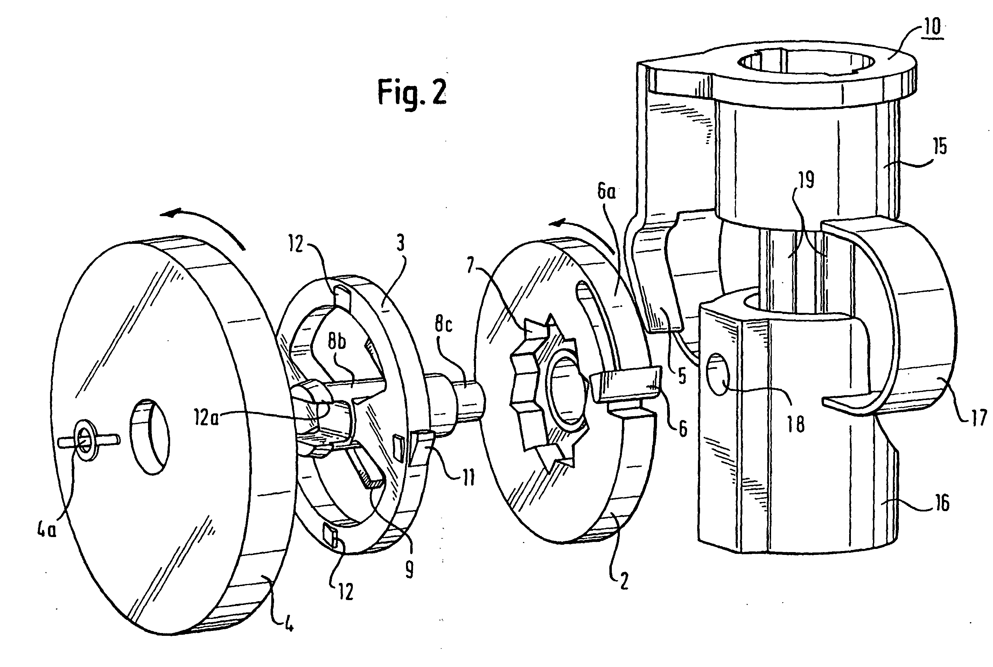

Zur Einleitung und für ein besseres Verständnis der Erfindung wird im folgenden zunächst ein Zählwerk für Dosieraerosolabgabevorrichtungen unter Bezugnahme auf Fig. 1 und 2 beschrieben. Fig. 1 und 2 zeigen jeweils aus zwei unterschiedlichen Blickrichtungen betrachtete Explosionsdarstellungen des Zählwerks.

Das Zählwerk weist im wesentlichen vier Scheibeneinheiten 1, 2, 3 und 4 auf, die parallel zueinander angeordnet sind. Die Mittelpunkte der Scheibeneinheiten 1, 2, 3 und 4 liegen auf einer zu den Scheibeneinheiten senkrecht stehenden Achse und sind, wie nachstehend noch genauer erläutert wird, über einen Lagerbolzen 8 mit einer Betätigungsvorrichtung 10 eines Dosieraerosolerzeugers (nicht dargestellt) verbunden. Die erste, zweite und vierte Scheibeneinheit 1, 2 und 4 sind bezogen auf die Betätigungsvorrichtung 10 drehbar angeordnet. Die dritte Scheibeneinheit 3 nimmt eine feste Position bezogen auf die Betätigungsvorrichtung 10 und damit auch auf die übrigen Scheibeneinheiten ein.

Die erste Scheibeneinheit 1 besitzt ein Mittel zur Aufnahme einer Kraft, die eine Drehung der ersten Scheibeneinheit hervorruft; dabei handelt es sich um eine Außenverzahnung 1a, in die ein an der Betätigungsvorrichtung 10 angebrachter Betätigungsarm 5 eingreift (siehe Fig. 1 und 2). Die erste Scheibeneinheit 1 weist einen kleineren Radius als die zweite Scheibeneinheit 2 auf und ist mit dieser fest verbunden. Durch diese feste Verbindung wird die Übertragung der Drehbewegung von der ersten Scheibeneinheit 1 auf die zweite Scheibeneinheit 2 realisiert.

Um die Drehbewegung an eine vierte Scheibeneinheit 4 zu übertragen, ist als Übertragungsmittel am Außenumfang der zweiten Scheibeneinheit 2 ein keilförmiger Nocken 6 angebracht, der über ein Federelement 6a mit der zweiten Scheibeneinheit 2 verbunden ist und der im folgenden als Federnocken bezeichnet wird. Das Federelement 6a bildet im wesentlichen die Außenkontur der zweiten Scheibeneinheit 2 nach, besitzt aber ein frei schwingendes Ende. An dem frei schwingendem Ende ist der keilförmige Federnocken 6 derart angebracht, daß er in der Ruhestellung über die Außenkontur der zweiten Scheibeneinheit 2 in radialer Richtung übersteht. Die Geometrie des Federnocken ist so gewählt, daß der Nocken bzw. die Nase bei einer Auslenkung in radialer Richtung in die Innenverzahnung 13 der vierten Scheibeneinheit 4 eingreift.

Dazu ist der keilförmige Federnocken 6 so angeordnet, daß die scharfe und die stumpfe Kante senkrecht zur Ebene der zweiten Scheibeneinheit 2 orientiert sind. Der Federnocken 6 weist ferner eine Breite auf, die größer ist als die Dicke der zweiten Scheibeneinheit 2, so daß sich der Federnocken 6 parallel zur Achse der Scheibeneinheiten zapfenartig in Richtung auf die dritte und vierte Scheibeneinheit 3 und 4 erstreckt.

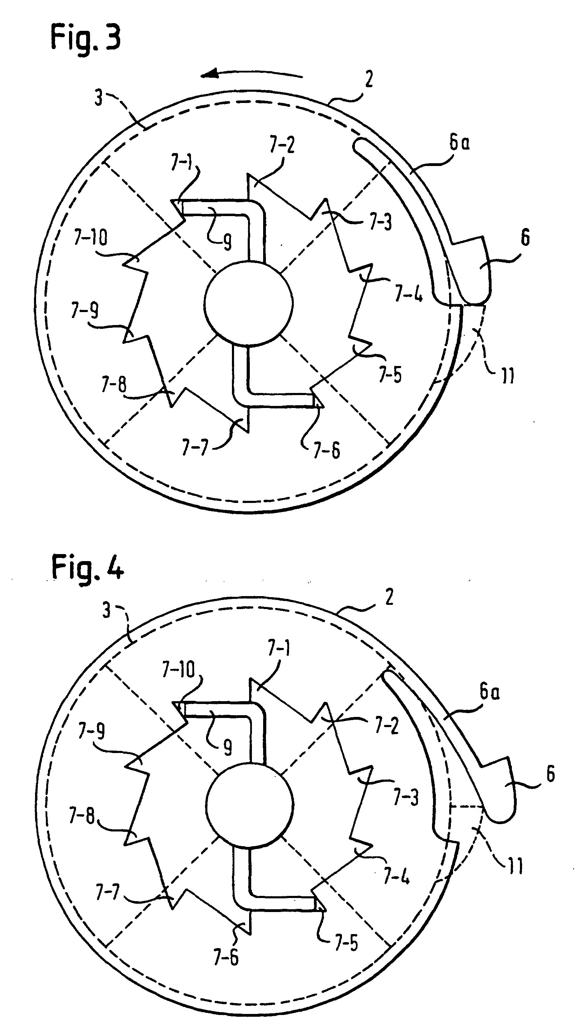

Der zapfenartig in Richtung dritte und vierte Scheibeneinheit überstehenden Federnocken 6 kann in Wechselwirkung mit den anderen Elementen des Zählwerks, die insbesondere unter Bezugnahme auf Fig. 3 und 4 genauer erläutert werden, mit einer ersten Verzahnung 13 der vierten Scheibeneinheit 4 in Eingriff treten (siehe Fig. 2).

Die in Richtung dritte und vierte Scheibeneinheit weisende Seite der zweiten Scheibeneinheit 2 weist eine Innenverzahnung 7 auf, die symmetrisch um die Achse der Scheibeneinheiten angeordnet ist. Die Innenverzahnung 7, die in Fig. 2 zu sehen ist, nicht aber in Fig.1, weist die gleiche Anzahl von Zähnen wie die außenverzahnte Scheibeneinheit 1 auf. In der in Fig. 1 bzw. 2 dargestellten Ausführungsform beträgt die Anzahl der Zähne zehn, um zusammen mit einer ersten Innenverzahnung 13 der vierten Scheibeneinheit 4 mit 24 Zähnen insgesamt 240 diskrete Zählschritte zu realisieren. Jedoch können problemlos andere Werte verwendet werden.

Der Radius der dritten Scheibeneinheit 3 ist gleich oder vorzugsweise geringfügig kleiner als der Radius der zweiten Scheibeneinheit 2. Ferner ist an dem Außenumfang der dritten Scheibeneinheit 3 eine keilförmige Schulter 11 angebracht, die zur Betätigung des an der zweiten Scheibeneinheit 2 angeordneten Federnocken 6 dient, was im folgenden noch genauer erklärt wird. Wird eine weitere Schulter angebracht, so ändert sich das Übersetztungsverhältnis. Die dritte Scheibeneinheit 3 ist mit einem Lagerbolzen 8 fest verbunden.

Der Lagerbolzen 8 weist bezogen auf die dritte Scheibeneinheit 3 drei unterschiedliche Bereiche auf, nämlich den in Richtung vierte Scheibeneinheit 4 weisenden Abschnitt 8a und die in Richtung erste und zweite Scheibeneinheit weisenden Abschnitte 8b und 8c. Der Durchmesser des Lagerbolzenabschnitts 8b ist mit dem des Abschnittes 8a identisch, und der des Abschnitts 8c ist im Vergleich dazu reduziert. Der Lagerbolzen 8 dient der drehfesten Anbringung der dritten Scheibeneinheit 3 an der Betätigungsvorrichtung 10 und zur drehbaren Lagerung der ersten, zweiten und vierten Scheibeneinheit 1, 2 und 4. Hierzu ist der Lagerbolzenabschnitt mit kleinerem Radius 8c in eine entsprechende Öffnung 18 der Befestigungseinheit 10 eingeführt und derart befestigt, daß die Position der dritten Scheibeneinheit 3 bezogen auf die Betätigungsvorrichtung 10 fest ist. Die in der ersten und zweiten Scheibeneinheiten 1 und 2 vorgesehenen Öffnungen sind so auf den Durchmesser des Lagerbolzenabschnittes 8b abgestimmt, daß die ersten und zweiten Scheibeneinheiten 1 und 2 auf dem Lagerbolzenabschnitt 8b gleitend drehbar sind. Es sei angemerkt, daß die Durchmesser der Abschnitte 8a, 8b und 8c nicht auf das in Fig. 1 bzw. Fig. 2 dargestellte Verhältnis beschränkt sind. Wesentlich ist, daß die jeweiligen Öffnungen der Scheibeneinheiten und die Durchmesser der Lagerbolzenabschnitte aufeinander abgestimmt sind.

Ferner sind an dem Lagerbolzen 8 zwei L-förmige Verriegelungselemente 9 angeordnet, die am Außenumfang des Lagerbolzenabschnitts 8b angeordnet sind und die in die Innenverzahnung 7 der zweiten Scheibeneinheit 2 eingreifen. Die Öffnung der vierten Scheibeneinheit 4 ist so auf den Durchmesser des Lagerbolzenabschnittes 8a abgestimmt, daß die vierte Scheibeneinheit 4 auf dem Lagerbolzenabschnitt 8a gleitend drehbar ist. An dem Abschnitt 8a sind zudem, in Richtung vierte Scheibeneinheit 4 weisend, zwei Schnapphaken 12a symmetrisch angeordnet, die zur axialen Fixierung der Scheibeneinheit 4 dienen.

Auf der in Richtung vierte Scheibeneinheit 4 weisenden Seite der dritten Scheibeneinheit 3 ist eine Rastverzahnung 12 angebracht, die in Fig. 2 zu sehen ist. In dem in Fig. 2 dargestellten Ausführungsbeispiel besteht die Rastverzahnung 12 aus vier Elementen, die um 90° zueinander beabstandet. sind. Die Rastverzahnung kann aber auch aus einem oder aus mehreren Elementen bestehen, die zentrisch um die Achse des Lagerbolzens 8 angeordnet sind. Diese Rastverzahnung 12 greift in eine zweite Verzahnung 14 der vierten Scheibeneinheit 4 ein und dient im wesentlichen der Sicherung der Drehstellung der Scheibeneinheit 4.

Die Ausgestaltung der vierten Scheibeneinheit 4 ist insbesondere aus Fig. 1 ersichtlich. Die in Richtung der anderen Scheibeneinheiten weisende Seite der vierten Scheibeneinheit 4 weist eine erste Verzahnung 13 auf, die an der Umfangsinnenseite angeordnet ist, und eine zweite Verzahnung 14, die kreisförmig um den Mittelpunkt der vierten Scheibeneinheit 4 verläuft. Der Radius der zweiten Verzahnung 14 ist abgestimmt auf die Lage der Elemente der Rastverzahnung 12, die an der dritten Scheibeneinheit 3 vorgesehen ist.

Im montierten Zustand sind die erste und zweite Scheibeneinheit 1 und 2 auf dem Lagerbolzenabschnitt 8b angeordnet; dabei liegen die L-förmigen Verrigerlungselemente 9 in der Ausnehmung der Innenverzahnung 7 der zweiten Scheibeneinheit 2. Der Federnocken 6 ragt über die Außenkontur der dritten Scheibeneinheit 3. Die vierte Scheibeneinheit 4 ist auf dem Lagerbolzenabschnitt 8a angeordnet und nimmt aufgrund der kappenartigen Gestalt die dritte Scheibeneinheit 3 auf; dabei liegt der Federnocken 6 zwischen der Außenkontur der dritten Scheibeneinheit 3 und der ersten Innenverzahnung der vierten Scheibeneinheit 4.

Im folgenden wird die Funktionsweise des zuvor beschriebenen Zählwerks erläutert. Da, wie in Fig. 2 gezeigt, der Betätigungsarm 5 der erfindungsgemäßen Betätigungseinrichtung 10 in die Außenverzahnung der ersten Scheibeneinheit 1 eingreift, wird durch eine Bewegung des Betätigungsarmes 5 die erste Scheibeneinheit 1 in die in Fig. 1 und 2 angedeutete Pfeilrichtung gedreht. Hierbei bewegt sich die zweite Scheibeneinheit 2 mit, da die erste und zweite Scheibeneinheit 1 und 2 fest miteinander verbunden sind. Die erste und zweite Scheibeneinheit 1 und 2 gleiten dabei auf dem Lagerbolzenabschnitt 8b. Im Gegensatz dazu ist die dritte Scheibeneinheit 3 über den Lagerbolzen 8 mit der Betätigungseinrichtung 10 fest verbunden, so daß die Position der dritten Scheibeneinheit 3 bezogen auf die Betätigungseinrichtung 10 zu jedem Zeitpunkt unverändert bleibt. Gleichzeitig sind die an dem Lagerbolzenabschnitt 8b angebrachten L-förmigen Verriegelungselemente 9 in Eingriff mit der Innenverzahnung 7 der Scheibeneinheit 2; jedoch gestattet die Gestaltung der Verriegelungselemente 9 und der Innenverzahnung 7 eine Drehung der ersten und zweiten Scheibeneinheiten 1 und 2 in der in Fig. 1 und 2 gezeigten Richtung. Bei einer Drehung der ersten und zweiten Scheibeneinheit 1 und 2 in Pfeilrichtung rasten die Verriegelungselemente 9 in die jeweils nächste Verzahnung der Innenverzahnung 7 ein. Bei einer erneuten Betätigung des Armes 5 drehen sich Scheibeneinheiten 1 und 2 genau einen Zahn weiter und der Verriegelungsarm rastet in den nächsten Zahn ein. Durch ein derartiges Eingreifen der Verriegelungselemente 9 in die Innenverzahnung 7 wird sichergestellt, daß sich die erste und somit auch die zweite Scheibeneinheit beim Lösen des Betätigungsarms 5 aus dem Eingriff mit der zahnradförmigen Scheibeneinheit 1 nicht zurückdreht, d.h. sich nicht entgegen der Pfeilrichtung zurückbewegt.

Bei der in Fig. 1 und 2 angedeueteten Drehung der ersten und zweiten Scheibeneinheit 1 und 2 in Pfeilrichtung läuft der an der zweiten Scheibeneinheit 2 angeordnete Federnocken 6 auf der Außenkontur der dritten Scheibeneinheit 3 entlang. Geht man von einem Zahnrad, das zehn Zähne aufweist aus, so überstreicht der Federnocken 6 bei 10-maliger Betätigung des Armes 5 einen Winkel von 360°, d.h. der Federnocken macht einen vollen Umlauf und gelangt wieder an seine Ausgangsposition. Solange der Federnocken 6 nicht von der Schulter 11 der dritten Scheibeneinheit 3 beeinflußt wird, greift der Federnocken 6 nicht in die an der Innenumfangseite der vierten Scheibeneinheit 4 angebrachte erste Verzahnung 13 ein. Erst wenn die Schulter 11 den Federnocken 6 in radialer Richtung anhebt, gelangt der Federnocken 6 in Eingriff mit der ersten Innenverzahnung der vierten Scheibeneinheit 4 und bewirkt eine Drehung der vierten Scheibeneinheit um eine Drehstellung.

Die schrittweise Drehung der Scheibeneinheit 2 bezogen auf die dritte Scheibeneinheit 3, sowie das Eingreifen des Federnockens 6 der zweiten Scheibeneinheit 2 in die Innenverzahnung 13 der vierten Scheibeneinheit 4 werden im folgenden unter Bezugnahme auf Fig. 3 und 4 genauer beschrieben. Fig. 3 und 4 zeigen schematische Querschnittsansichten, die insbesondere das Zusammenwirken von zweiter und dritter Scheibeneinheit 2 und 3 detaillierter erläutern. In Fig. 3 und 4 sind die Scheibeneinheiten 2 und 3 aus der in Fig. 2 gezeigten Blickrichtung dargestellt. Die dritte Scheibeneinheit 3 ist in beiden Abbildungen aus Gründen der besseren Übersicht lediglich gestrichelt angedeutet. Fig. 3 zeigt die oben erwähnte Ausgangsposition bzw. Ruheposition der zweiten Scheibeneinheit 2 in Bezug auf die fest mit der Betätigungseinrichtung 10 verbundene dritte Scheibeneinheit 3. In dieser Position ist das Verriegelungselement 9 in den ersten Zahn 7-1 der Innenverzahnung 7 eingerastet. Durch Betätigung des Betätigungsarmes 5, der in die zahnradförmige erste Scheibeneinheit 1 eingreift (in Fig. 3 nicht dargestellt), wird die Scheibeneinheit 2 schrittweise, d.h. Zahn für Zahn, in der in Fig. 3 angedeuteten Pfeilrichtung gedreht. Das Verriegelungselement 9 rastet hierbei, beginnend vom ersten Innenzahn 7-1 in aufsteigender Reihenfolge in die Zähne 7-2 bis 7-9 ein. Bei der durch den Betätigungsarm 5 initiierten Drehbewegung der ersten und zweiten Scheibeneinheit 1 und 2 läuft der Federnocken 6 auf dem Umfang der in Fig. 3 gestrichelt dargestellten dritten Scheibeneinheit 3 entlang, ohne daß der Federnocken 6 ausgelenkt wird. In diesem Fall greift der Federnocken 6 nicht in die erste Verzahnung 13 der vierten Scheibeneinheit 4 ein.

Ist der Betätigungsarm 5 so oft betätigt worden, daß das Verriegelungselement 9 in den Zahn 7-10 der Innenverzahnung 7 einrastet, d.h wenn ausgehend von der in Fig. 3 dargestellten Ausgangsposition der Betätigungsarm 5 zum neunten Mal betätigt worden ist, dann ist der Federnocken 6 in Kontakt mit der Schulter 11. Dies bewirkt, daß der Federnocken 6, wie in Fig. 4 dargestellt, in radialer Richtung ausgelenkt wird. Mit anderen Worten, der Federnocken 6 wird in dieser Position durch die Schulter 11 betätigt und der Federnocken 6 greift in die Innenumfangsverzahnung 13 ein und dreht die vierte Scheibeneinheit 4 um einen Zahn der ersten Verzahnung 13 in Pfeilrichtung weiter.

Bei dieser durch das Zusammenwirken der Schulter 11 und des Federnocken 6 bewirkten Drehung der vierten Scheibeneinheit 4 rastet die an der in Richtung erste, zweite und dritte Scheibeneinheit weisenden Innenseite der vierten Scheibeneinheit 4 angeordnete zweite Verzahnung 14 mit der Rastverzahnung 12 ein. Die Rastverzahnung 12 und die zweite Verzahnung 14 der vierten Scheibeneinheit 4 dienen hierbei der Sicherung der vierten Scheibeneinheit 4 gegen Verdrehen, und durch die Schnapphaken 12a ist eine axiale Fixierung der vierten Scheibeneinheit 4 gewährleistet.

Bei der nachfolgenden Betätigung der ersten bzw. zweiten Scheibeneinheit durch den Betätigungsarm 5 nimmt die zweite Scheibeneinheit 2 wieder die in Fig. 3 dargestellte Ausgangsposition ein und die zuvor unter Bezugnahme auf Fig. 3 beschriebene Bewegung des Federnocken 6 wird wieder durchlaufen, ohne daß der Federnocken 6 in Eingriff mit der ersten Verzahnung 13 der vierten Scheibeneinheit 4 tritt. Erst nachdem das Verriegelungselement wieder in den Zahn 7-10 der Innenverzahnung 7 einrastet, wird der Federnocken 6 durch die Schulter 11 erneut ausgelenkt, so daß dieser wieder in Eingriff mit der Innenverzahnung 13 tritt und daraufhin die vierte Scheibeneinheit 4 um einen Zahn der ersten Innenverzahnung 13 weiterdreht. Auf diese Weise wird bei einer 10-zahnigen ersten Scheibeneinheit die vierte Scheibeneinheit 4 jeweils nach 10 Betätigungen des Betätigungsarms 5 um genau einen Zahn der ersten Verzahnung 13 weitergedreht. Wenn die erste Innenverzahnung 13 der vierten Scheibeneinheit 4 beispielsweise 24 Zähne aufweist, beträgt die maximale Anzahl der Zählstellungen in diesem Fall 240, d.h. wenn sich die vierte Scheibeneinheit 4 um 360° gedreht hat, sind 240 Betätigungen des Betätigungsarms 5 gezählt worden. Die zweite Verzahnung 14 der vierten Scheibeneinheit 4 weist die gleiche Anzahl von Zähnen wie die erste Innenverzahnung 13 auf.

Es sei angemerkt, daß die Anzahl der Zähne der ersten und zweiten Verzahnung 13 und 14 entsprechend an die gewünschte Übersetzung angepaßt werden kann. Eine 30-zahnige erste bzw. zweite Verzahnung 13 bzw. 14 dient somit zur Zählung von bis zu 300 Betätigungen. Das Übersetzungsverhältnis des erfindungsgemäßen Zählwerks bzw. die Anzahl der Zählungen kann aber auch durch ein Anordnen einer weiteren Schulter bzw. mehrerer Schultern am Außenrand der dritten Scheibeneinheit 3 variiert werden. Dies ermöglicht insbesondere ein unkompliziertes und schnelles Anpassen des erfindungsgemäßen Zählwerks an ein gewünschtes Übersetzungsverhältnis.

Die vierte Scheibeneinheit 4 kann in Form einer Anzeigescheibe ausgebildet sein, um entsprechend die Zählungen anzuzeigen. Dies kann beispielsweise durch einen Aufdruck eines Zeigers oder einer Farbmarkierung sowohl auf der Stirnseite als auch auf dem Rand der vierten Scheibeneinheit 4 erreicht werden. In dem in Fig. 2 dargestellten Ausführungsbeispiel ist ein Zeiger-Anzeigeelement 4a angedeutet. Der Zeiger gibt entsprechend der Drehstellung der vierten Scheibeneinheit 4 die Anzahl der Betätigungen an.

Im folgenden wird die Betätigungseinrichtung 10 gemäß der Erfindung beispielhaft genauer erläutert, die in vorteilhafter Weise mit dem zuvor beschriebenen Zählwerk ggf. aber auch mit anderen Zählwerken zusammenarbeitet.

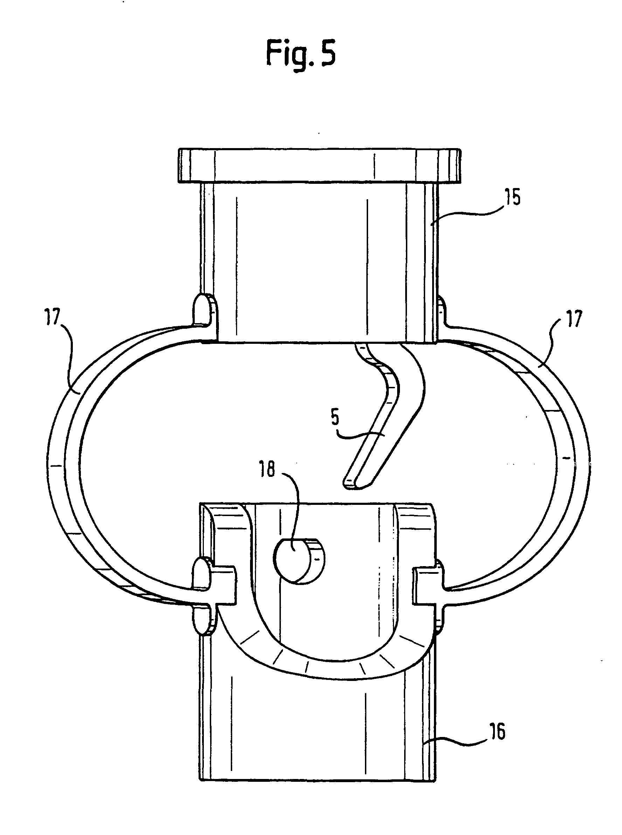

Die in Fig. 1 und 2 dargestellte Betätigungseinrichtung 10 besteht aus einer zylinderförmigen Hülse 15 und einem zylinderförmigen Unterteil 16, bei dem es sich ebenfalls um eine Hülse handelt. An der Außenseite der Hülse 15 ist ein Betätigungsarm 5 angebracht. Hülse 15 und Unterteil 16 sind über eine bogenförmigen Feder 17 miteinander verbunden. Bei Betätigung der Feder 17, d.h. beim Zusammendrücken von Hülse 15 und Unterteil 16 in axialer Richtung, wird durch geeignete Führungselemente 19, die an der Hülse 15 in Richtung Unterteil 16 weisend angebracht sind, eine geradlinige Bewegung der Hülse 15 bezogen auf des Unterteil 16 gewährleistet. Dabei gleiten die Innenumfangsseite der Hülse 15 auf der Außenseite der Führungselemente 19. Wesentlich ist, daß eine geradlinige Bewegung der Hülse 15 und somit auch eine geradlinige Bewegung des Betätigungsarms 5 beim Zusammendrücken der Betätigungseinrichtung 10 gewährleistet ist, und ein zuverlässiges Eingreifen des Betätigungsarmes 5 in die zahnradförmige erste Scheibeneinheit 1 erzielt wird. Durch die Gestaltung der bogenförmigen Feder 17, beispielsweise wie in Fig. 1 und 2 gezeigt, als flaches gebogenes Federelement kann eine geradlinige Bewegung der Hülse 15 in Bezug auf das Unterteil erreicht werden, ohne daß Führungselemente erforderlich sind. Zur Verdeutlichung ist eine entsprechende Gestaltung der Betätigungseinrichtung 10 in Fig. 5 dargestellt, wobei das Zählwerk in der Darstellung der Fig. 5 weggelassen wurde.

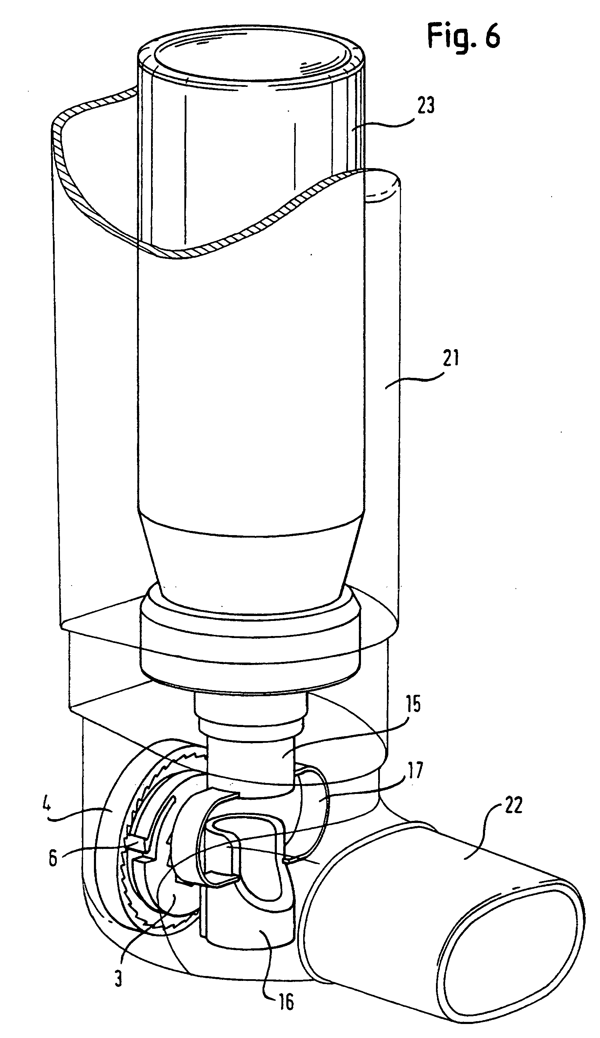

In Fig. 6 ist in einer Gesamtansicht die Anordnung der erfindungsgemäßen Betätigungseinrichtung und des zuvor beschriebenen Zählwerks in einer Dosieraerosolabgabevorrichtung dargestellt. Fig. 6 zeigt ein Gehäuse 21 der Dosieraerosolabgabevorrichtung, das mit einem Mundstück 22 versehen ist. Das Mundstück 22 ist an dem unteren Bereich des Gehäuses 21 in abgewinkelter Stellung angeordnet. Im oberen Bereich des Gehäuses 21 befindet sich ein Dosieraerosolbehälter 23 zur Aufnahme eines zu zerstäubenden Aerosols. An den unteren Bereich des Aerosolbehälters 23 schließt sich ein Düsenbereich an, der in die Führungshülse 15 der Betätigungsvorrichtung 10 eingeführt ist. Die Düse selbst ist zur Verdeutlichung der Lage der erfindungsgemäßen Betätigungseinrichtung in Fig. 6 nicht dargestellt. Die Betätigungsvorrichtung 10 ist mit dem erfindungsgemäßen Zählwerk, bestehend aus den Scheibeneinheiten 1, 2 , 3 und 4, verbunden, um die abgegebenen bzw. noch verbleibenden Dosierungen zu zählen und anzuzeigen. Bei jedem Pumpstoß, der, wie zuvor beschrieben, durch die Betätigung der Dosieraerosolabgabevorrichtung ausgelöst wird, wird eine bestimmte Aerosolmenge ausgegeben und über eine Düse zerstäubt, deren Aufbau und Verwendung mit derartigen herkömmlichen Dosieraerosolabgabevorrichtungen allgemein bekannt ist. Das erzeugte Aerosol kann vo einem Patienten über das Mundstück 22 eingeatmet werden. Die Betätigung der Dosieraerosolvorrichtung bewirkt das sich die Führungshülse 15 zusammen mit dem Betätigungsarm der Betätigungsvorrichtung 10 sich gegenüber dem Unterteil 16 verschiebt. Dadurch führt die Betätigung der Dosieraerosolabgabevorrichtung zu einer Weiterschaltung des Zählwerks.

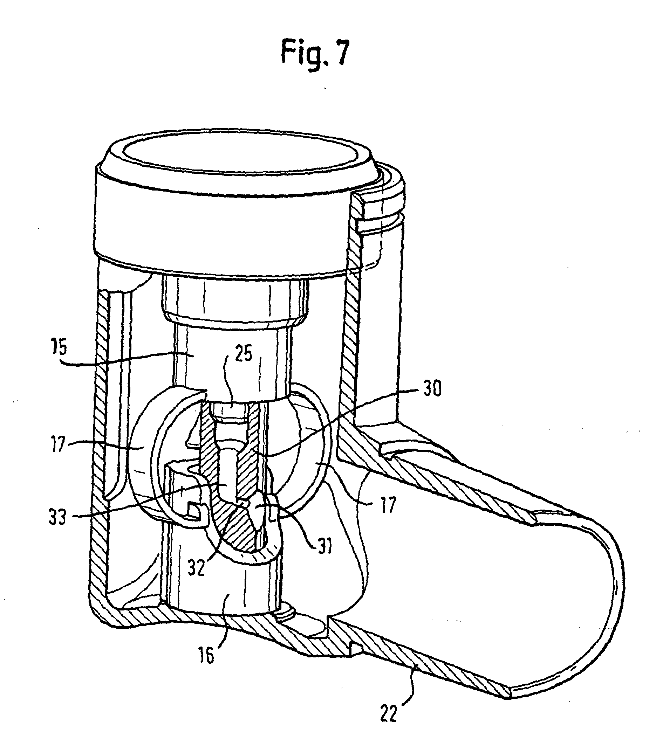

Wie aus Fig. 6 hervorgeht, kann das Zählwerk mit der erfindungsgemäßen Betätigungseinrichtung unterhalb des Aerosolbehälters in einer herkömmlichen Dosieraerosolausgabevorrichtung problemlos angeordnet werden. Dies wird vorrangig dadurch erreicht, daß, wie schon aus Fig. 6 entnehmbar ist, sowohl die Hülse 15 als auch das Unterteil 16 der Betätigungsvorrichtung 10 im Innern Raum für insbesondere den Anschlußstutzen (nicht sichtbar) des Aerosolbehälters 23 und für den Düsenkörper der Dosieraerosolabgabevorrichtung aufweist. In Fig. 7 ist dargestellt, wie ein zylindrischer Düsenkörper 30 der Dosieraerosolabgabevorrichtung 21 in der Hülse 15 und dem Unterteil 16 der Betätigungseinrichtung 10 angeordnet werden kann. Man erkennt den (geschnitten dargestellten) zylindrischen Düsenkörper 30, die trichterförmige Düsenöffnung 31, einen Verbindungskanal 32, der die trichterförmige Düsenöffnung 31 mit einer Düsenkammer 33 verbindet, in die der Anschlußstutzen 25 des Aerosolbehältes 23 das Aerosol bei einem Pumpstoß abgibt. Da erfindungsgemäß die Hülse 15 und das Unterteil 16 der Betätigungseinrichtung 10 im Inneren ausreichend Raum sowohl für die Düse 30 als auch für den Anschluß des Aerosolbehältes 23 zur Verfügung stellen und da das Unterteil 16 so gestaltet ist, dass es die Düse 31 vollständig freigibt und die Aerosolerzeugung und -ausbreitung nicht beeinflusst, kann die Betätigungseinrichtung 10 problemlos am Düsenkörper einer gegebenen Dosieraerosolabgabevorrichtung angeordnet werden, ohne daß Änderungen an dem Düsenkörper 30 bzw. der Düse 31, 32 oder dem Anschluß 25 für den Aerosolbehälter vorgenommen werden müssen. Das bedeutet, daß ein für die Dosieraerosolabgabevorrichtung durchlaufendes Zulassungsverfahren nicht erneut erforderlich ist, da keine Modifikationen an der Düsengestaltung 30, 31, 32, 33 aufgrund der erfindungsgemäßen Betätigungseinrichtung 10 vorgenommen werden müssen.

Die Freigabe der Düse 31 wird bei dem gezeigten Beispiel einer erfindungsgemäßen Betätigungseinrichtung dadurch erreicht, dass die das Untereil 16 bildende Hülse ein der der Düse zugewandten Seite eine entsprechende Aussparung aufweist, wie die Fig. 1, 2, 5, 6 und 7 zeigen. Alternativ kann auch eine Öffnung vorgesehen werden, die der Öffnung 8 ähnelt, die aber so ausgebildet ist, dass sie die Düse 31 freigibt .

Vorteilhaft ist somit, daß beim Einsatz eines Zählwerks mit einer erfindungsgemäßen Betätigungseinrichtung 10 eine herkömmliche Dosieraerosolabgabevorrichtung nicht in ihren wesentlichen Komponenten, insbesondere nicht die Düse, verändert werden muß. Durch eine einfache Anordnung einer Betätigungsvorrichtung zum Betätigen des Zählwerks in einer gegebenen Gerätegestaltung wird ein einfacher und unkomplizierter Aufbau ermöglicht, der universell mit bereits bekannten Geräten verwendet werden kann. Auf diese Weise kann auf eine aufwendige Modifikation der bereits bekannten Dosieraerosolabgabevorrichtunen verzichtet werden. Dies ist wesentlich von Vorteil, da beispielsweise bei einer Änderung der Düsenform derartige Inhaltoren erneut medizinische Prüfungen zur Erlangung der Zulassung durchlaufen müßten, die in der Regel langwierig, aufwendig und somit kostspielig sind.

## Patentansprüche

1. Dosieraerosolabgabevorrichtung mit einem Aerosolbehälter (23), einer in einem Düsenkörper(30) angeordneten Düse (31, 32), über die ein Aerosol abgegeben wird, wenn der Aerosolbehälter (23) gegenüber dem Düsenkörper (30) bei einer Betätigung der Dosieraerosolabgabevorrichtung verschoben wird, einem Zählwerk (1, 2, 3, 4) zur Zählung der Betätigungen der Dosieraerosolabgabevorrichtung und einer Betätigungsvorrichtung (10) zur Weiterschaltung des Zählwerks (1, 2, 3, 4) bei einer Betätigung der Dosieraerosolabgabevorrichtung,
**dadurch gekennzeichnet, dass**
die Betätigungsvorrichtung (10) zwei gegeneinander verschiebbare Hülsen (15, 16) umfasst, die im Inneren den Düsenkörper (30) der Dosieraerosolabgabevorrichtung derart aufnehmen, dass die Düse (31) zur unbeeinflussten Aerosolerzeugung freigegeben ist, die geradlinig gegeneinander verschiebbar sind und die durch Federelemente (17) miteinander verbunden sind, die die Hülsen (15, 16) in eine Ausgangsposition zurück bewegen.

2. Dosieraerosolabgabevorrichtung nach Anspruch 1,
**dadurch gekennzeichnet, dass**
die Federelemente (17) in Form von flachen, gebogenen Federelementen realisiert sind, die an den Außenseiten der Hülsen (15, 16) befestigt oder einstückig angeformt sind.

3. Dosieraerosolabgabevorrichtung nach einem der Ansprüche 1 oder 2,
**dadurch gekennzeichnet, dass**
an den Hülsen (15, 16) der Betätigungsvorrichtung (10) Führungselemente (19) vorgesehen sind, die eine geradlinige Verschiebung der beiden Hülsen (15, 16) gegeneinander gewährleisten.

4. Dosieraerosolabgabevorrichtung nach Ansprüche 1 oder 2,
**dadurch gekennzeichnet, dass**
die Federelemente (17) derart ausgestaltet sind, dass sie eine geradlinige Verschiebung der beiden Hülsen (15, 16) gegeneinander gewährleisten.

5. Dosieraerosolabgabevorrichtung nach einem der Ansprüche 1 bis 4,
**dadurch gekennzeichnet, dass**
die Betätigungsvorrichtung (10) einen Betätigungsarm (5) umfasst, der auf das Zählwerk einwirkt, um das Zählwerk weiterzuschalten.

6. Dosieraerosolabgabevorrichtung nach einem der Ansprüche 1 bis 5,
**dadurch gekennzeichnet, dass**
das Zählwerk aus mehreren parallel angeordneten Scheibeneinheiten (1, 2, 3, 4) aufgebaut ist und eine erste Scheibeneinheit eine Außenverzahnung (1a) aufweist, die mit der Betätigungsvorrichtung (10) zusammenwirkt.

7. Dosieraerosolabgabevorrichtung nach einem der Ansprüche 1 bis 6,
**dadurch gekennzeichnet, dass**
die Betätigungsvorrichtung (10) eine Öffnung (18) aufweist, in der ein Lagerbolzen (8c) des Zählwerks zur Halterung des Zählwerks an der Betätigungsvorrichtung (10) befestigt ist.

8. Dosieraerosolabgabevorrichtung nach einem der Ansprüche 1 bis 7,
**dadurch gekennzeichnet, dass**
die Betätigungseinrichtung (10) eine Aussparung oder eine Öffnung aufweist, durch die die Düse (31) freigegeben ist.

## Claims

1. Metering aerosol delivery device having an aerosol vessel (23), a nozzle (31, 32) arranged in a nozzle member (30) through which an aerosol is delivered when the aerosol vessel (23) is displaced relative to the nozzle member (30) on actuation of the metering aerosol delivery device, a counting mechanism (1, 2, 3, 4) for counting the actuations of the metering aerosol delivery device and actuating device (10) for incrementing the counting mechanism (1, 2, 3, 4) on actuation of the metering aerosol delivery device,
**characterised in that**
the actuating device (10) comprises two sleeves (15, 16) displaceable relative to one another which in the interior accommodate the nozzle member (30) of the metering aerosol delivery device in such a way that the nozzle (31) has clearance for unimpeded aerosol generation, which sleeves are displaceable relative to one another in rectilinear manner and which are connected to one another by spring elements (17) which move the sleeves (15, 16) back into a starting position.

2. Metering aerosol delivery device according to claim 1, **characterised in that** the spring elements (17) are implemented in the form of flat, bent spring elements which are fastened or moulded in place in one piece on the outsides of the sleeves.

3. Metering aerosol delivery device according to one of claims 1 or 2, **characterised in that** on the sleeves (15, 16) of the actuating device (10) guide elements (19) are provided which ensure rectilinear displacement of the two sleeves (15, 16) relative to one another.

4. Metering aerosol delivery device according to claims 1 or 2, **characterised in that** the spring elements (17) are designed in such a way that they ensure rectilinear displacement of the two sleeves (15, 16) relative to one another.

5. Metering aerosol delivery device according to one of claims 1 to 4, **characterised in that** the actuating device (10) comprises an actuating arm (5) which acts on the counting mechanism in order to increment the counting mechanism.

6. Metering aerosol delivery device according to one of claims 1 to 5, **characterised in that** the counting mechanism is built up of a plurality of disk units (1, 2, 3, 4) arranged in parallel and a first disk unit has an outer toothed structure (1a) which acts in conjunction with the actuating device (10).

7. Metering aerosol delivery device according to one of claims 1 to 6, **characterised in that** the actuating device (10) has an aperture (18) in which a bearing pin (8c) of the counting mechanism is fastened for mounting the counting mechanism on the actuating device (10).

8. Metering aerosol delivery device according to one of claims 1 to 7, **characterised in that** the actuating device (10) has a recess or opening through which the nozzle (31) is given clearance.

## Revendications

1. Dispositif de délivrance d'un aérosol dosé, avec un réservoir à aérosol (23), une buse (31, 32) disposée dans un corps de buse (30), par l'intermédiaire de laquelle un aérosol est délivré, lorsque le réservoir à aérosol (23) est déplacé par rapport au corps de buse (30), lors d'un actionnement du dispositif de délivrance d'aérosol dosé, un mécanisme de comptage (1, 2, 3, 4), pour compter les actionnements du dispositif de délivrance d'aérosol dosé, et un dispositif d'actionnement (10) pour continuer la manoeuvre du compteur (1, 2, 3, 4) en cas d'actionnement du dispositif de délivrance d'aérosol dosé,
**caractérisé en ce que**
le dispositif d'actionnement (10) comprend deux douilles (15, 16), coulissantes l'une par rapport à l'autre, qui reçoivent intérieurement le corps de buse (30). du dispositif de délivrance d'aérosol dosé, de manière que la buse (31) soit libérée pour générer un aérosol de façon non-influencée, par le fait que les douilles sont déplaçables l'une par rapport à l'autre en ligne droite et reliées ensemble par des éléments élastiques (17), qui ramènent à les douilles (15, 16) une position initiale.

2. Dispositif de délivrance d'un aérosol dosé selon la revendication 1, **caractérisé en ce que** les éléments élastiques (17) sont réalisés sous la forme d'éléments de ressort incurvés ou pliés, plats, fixés ou formés d'un seul tenant, sur les faces extérieures des douilles (15; 16).

3. Dispositif de délivrance d'un aérosol dosé selon l'une des revendications 1 ou 2, **caractérisé en ce que**, sur les douilles (15, 16) du dispositif d'actionnement (10), sont prévus des éléments de guidage (19), assurant un coulissement, rectiligne l'une par rapport l'autre, des deux douilles (15, 16).

4. Dispositif de délivrance d'un aérosol dosé selon la revendication 1 ou 2, **caractérisé en ce que** les éléments élastiques (17) sont réalisés de manière à assurer un coulissement, rectiligne l'une par rapport à l'autre, des deux douilles (15, 16).

5. Dispositif de délivrance d'un aérosol dosé selon l'une des revendications 1 à 4, **caractérisé en ce que** le dispositif d'actionnement (10) comprend un bras d'actionnement (5), agissant sur le mécanisme de comptage, pour continuer la manoeuvre du compteur.

6. Dispositif de délivrance d'un aérosol dosé selon l'une des revendications 1 à 5, **caractérisé en ce que** le mécanisme de comptage est formé d'une pluralité d'unités à disque (1, 2, 3, 4), disposées en parallèle, et une première unité à disque présente une denture extérieure (1a), coopérant avec le dispositif d'actionnement (10).

7. Dispositif de délivrance d'un aérosol dosé selon l'une des revendications 1 à 6, **caractérisé en ce que** le dispositif d'actionnement (10) présente une ouverture (18), dans laquelle est fixé un boulon de palier (8c) du mécanisme de comptage, pour la fixation du mécanisme de comptage sur le dispositif d'actionnement (10).

8. Dispositif de délivrance d'un aérosol dosé selon l'une des revendications 1 à 7, **caractérisé en ce que** le dispositif d'actionnement (10) présente un évidement ou une ouverture, à travers laquelle la buse (31) est exposée.
